(19) Europäisches Patentamt
European Patent Office
Office européen des brevets

(11) Numéro de publication: **0 140 400**
**A1**

(12) **DEMANDE DE BREVET EUROPEEN**

(21) Numéro de dépôt: **84201167.8**

(22) Date de dépôt: **13.08.84**

(51) Int. Cl.[4]: **C 07 B 41/12**
C 07 D 307/54, C 07 D 307/42
C 07 D 307/71, C 07 C 67/343

(30) Priorité: **30.09.83 FR 8315753**

(43) Date de publication de la demande:
**08.05.85 Bulletin 85/19**

(84) Etats contractants désignés:
**AT BE CH DE GB IT LI LU NL SE**

(71) Demandeur: **AGRIFURANE S.A.**
**Z.I. de Boé Cidex 4412**
**F-47240 Bon Encontre(FR)**

(72) Inventeur: **Delmas, Michel**
**23 résidence des Amandiers Deyme**
**F-31450 Montgiscard(FR)**

(72) Inventeur: **Gaset, Antoine**
**75 allée de Brienne**
**F-31000 Toulouse(FR)**

(72) Inventeur: **Le Bigot, Yves**
**Mas de Roux Bragassargues**
**F-30260 Quissac(FR)**

(74) Mandataire: **Barre, Philippe**
**Cabinet Barre-Gatti-Laforgue 95 rue des Amidonniers**
**F-31069 Toulouse Cédex(FR)**

(54) **Procédé de transformation directe d'un aldéhyde en un ester éthylénique.**

(57) L'invention concerne un procédé de transformation directe d'un aldéhyde en un ester éthylénique, du type dans lequel l'aldéhyde est mis en présence d'un phosphonate et d'un carbonate ou bicarbonate alcalin dans un solvant comportant une fonction alcool. Selon l'invention, on utilise un phosphonate de méthyle ou d'éthyle et, comme solvant, un alcool ou un polyol comportant un radical hydrocarboné autre que le méthyle ou éthyle, ce radical étant celui de l'ester éthylénique à obtenir. Le procédé de l'invention permet de réaliser la transformation directe d'aldéhyde furannique ou tétrahydrofurannique pour obtenir un ester éthylénique possédant un cycle furannique ou tétrahydrofurannique.

EP 0 140 400 A1

# PROCEDE DE TRANSFORMATION DIRECTE D'UN ALDEHYDE EN UN ESTER ETHYLENIQUE

L'invention concerne un procédé de transformation directe d'un aldéhyde en un ester éthylénique ; elle s'applique en particulier pour la transformation d'aldéhydes furanniques ou tétrahydrofuranniques.

On sait que la réaction de WITTIG-HORNER est actuellement utilisée pour obtenir, à partir d'aldéhydes, des alcènes et introduire sur la double liaison éthylénique des fonctions acides, esters, nitriles... Cette réaction en vue d'obtenir un ester éthylénique consiste à mettre en présence l'aldéhyde de départ avec les réactifs suivants : phosphonate et base et ce, dans un solvant organique inerte (J. SEYDENPENNE et coll., Tetrahedron, 1972, p. 4209 ; Tetrahedron, 1973, p. 2437 et Journal of American Chemical Society, 1980, p. 1270; C. PIECHUCKI, Synthesis, 1974, 869 ; M. MIKOLAJCZYK et coll., Synthesis, 1976, p. 396 ; J. KOVAC et coll., Collection Czechoslovakia Chemical Communications, 1976, p. 764 ; E. BREUER et coll., Tetrahedron, 1978, p. 997 ; A. FOUCAUD et Coll., Tetrahedron Letters, 1980, p. 2161 et Synthesis, 1979, p. 884 ; J. VILLIERAS et Coll.,Synthesis, 1982, p. 924 ; Synthesis, 1983, p. 300 et Phosphorus and Sulfur, 1983, p. 385). Un procédé amélioré est décrit dans la demande de brevet française n° 81.17825 au nom du demandeur ; ce procédé utilise un alcool comme solvant.

Dans tous ces procédés, la base permet d'arracher un proton sur le carbone en $\alpha$ du phosphore ; l'entité réactive ainsi formée se condense avec la fonction carbonyle de l'aldéhyde pour conduire à un oxy-anion intermédiaire qui évolue ensuite vers l'ester éthylénique. Le solvant organique ne constitue pas, à proprement parlé, un réactif de la réaction et n'est pas consommé dans celle-ci, même si dans le procédé de la demande n° 81.17825, ce solvant améliore le rendement en un type d'alcène recherché.

Les réactions ci-dessus évoquées conduisent nécessairement à un ester éthylénique dont les radicaux proviennent du phosphonate. Or actuellement peu de phosphonates sont disponibles commercialement et l'on trouve en fait sur le

marché, à faibles coûts, uniquement les phosphonates de méthyle ou d'éthyle. De ce fait, seuls les esters éthyléniques de méthyle ou d'éthyle de formule :

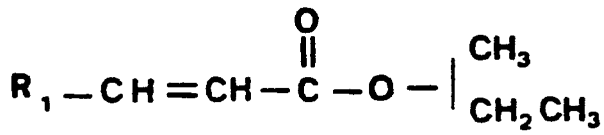

ont été synthétisés par la réaction de WITTIG-HORNER et réactions dérivées.

Bien entendu, il est toujours possible de substituer ensuite le radical méthyle ou éthyle par un nouveau radical souhaité, par une réaction de transestérification. Toutefois, cette double réaction exige une première extraction de l'ester éthylénique de méthyle ou éthyle au terme de la réaction de WITTIG-HORNER puis une seconde extraction visant à isoler l'ester éthylénique désiré. Outre les contraintes provenant de la mise en oeuvre de deux réactions successives, cette procédure conduit à un abaissement notable des rendements puisque le rendement global est le produit des rendements respectifs des deux réactions et des deux extractions.

La présente invention se propose d'éliminer les limitations sus-évoquées de la réaction de WITTIG-HORNER, en fournissant un procédé permettant de transformer, directement en une seule opération n'exigeant aucune séparation intermédiaire, un aldéhyde en un ester éthylénique de formule :

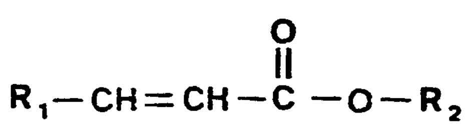

dans lequel $R_2$ est un radical hydrocarboné au choix de l'opérateur, autre que méthyle ou éthyle.

Un autre objectif de l'invention est de fournir un procédé de transformation permettant d'obtenir un rendement très élevé en ester éthylénique.

A cet effet, le procédé visé par l'invention est du type dans lequel un aldéhyde est mis en présence d'un phosphonate et d'un carbonate ou bicarbonate alcalin dans un solvant comportant une fonction alcool ; selon la présente invention, en vue d'obtenir un ester éthylénique

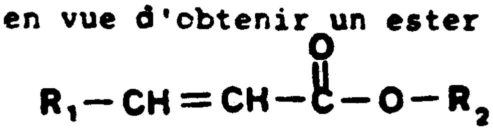

où $R_1$ est un hydrogène ou un radical ou cycle hydrocarboné provenant de l'aldéhyde de départ, et $R_2$ un radical hydrocarboné autre que méthyle ou éthyle, on utilise :

. d'une part, un phosphonate de méthyle ou d'éthyle

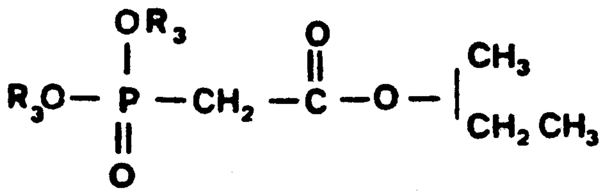

dans lequel $R_3$ est un radical ou un cycle hydrocarboné,

. d'autre part, un alcool ou un polyol $R_2OH$ comportant le radical hydrocarboné $R_2$ autre que méthyle ou éthyle, de l'ester éthylénique à obtenir.

Les inventeurs ont mis en évidence le fait inattendu que, d'une part, l'alcool ou le polyol, jusque-là limité à un simple rôle de solvant, peut jouer à la fois un rôle de solvant et de réactif en le choisissant avec un radical différent de celui du phosphonate, et que, d'autre part, dans les conditions ci-dessus évoquées, le carbonate ou le bicarbonate alcalin joue, outre son rôle classique de réactif dans la réaction de WITTIG-HORNER, un rôle de catalyseur dans l'échange des radicaux méthyle et éthyle par le radical $R_2$ désiré.

Le procédé de l'invention utilise ces deux phénomènes (sans qu'il ait été possible de déterminer s'ils sont simultanés ou successifs) et permet d'obtenir des esters

éthyléniques très diversifiés dans d'excellentes conditions de rendement et de sélectivité comme l'ont démontré les expérimentations ; ce résultat remarquable provient de la conjugaison inhabituelle des comportements suivants :

. réactif/catalyseur pour le carbonate et le bicarbonate,

. réactif/solvant pour l'alcool et le polyol.

Pour favoriser ces doubles comportements, le procédé est avantageusement mis en oeuvre dans les conditions suivantes :

- le carbonate ou bicarbonate alcalin est mis en oeuvre en quantité telle que la proportion molaire de celui-ci par rapport à l'aldéhyde est supérieure à 1,

- l'alcool ou le polyol est mis en oeuvre en quantité telle que la proportion molaire de celui-ci par rapport à l'aldéhyde est supérieure à 2.

Ainsi, les quantités des deux corps sus-évoqués sont adaptées pour leur permettre de jouer pleinement leur double rôle (c'est-à-dire avec un bon rendement pour chaque phénomène mis en jeu).

En outre, dans le but d'accroître encore le rendement, tout en bénéficiant de conditions faciles de mise en oeuvre, on limite le taux d'hydratation du milieu réactionnel à une valeur comprise entre environ 0,1 et 5 moles d'eau par mole d'aldéhyde.

Les phosphonates de méthyle ou d'éthyle les plus répandus et les moins onéreux sont ceux dans lesquels le radical $R_3$ est également un radical méthyle ou éthyle. Pour des raisons économiques, on choisira de préférence ce type de radical $R_3$.

La réaction de transformation directe ci-dessus évoquée peut être réalisée à la pression atmosphérique.

Il est également possible, et ceci améliore sensiblement le rendement, de travailler à pression réduite ; en pratique, l'on pourra prévoir dans ce cas une pression comprise entre 10 et 200 mm Hg (1 315 et 26 300 pascals).

L'alcool utilisé peut être un polyol dans lequel le radical $R_2$ contient entre 1 et 5 fonctions alcool, dans le but d'obtenir ~~des~~ esters éthyléniques hydroxylés. On peut également

utiliser un alcool gras possédant un radical $R_2$ à longue chaîne à nombre de carbones ⩾ 8, en vue d'obtenir un ester éthylénique comportant une chaîne lipophile.

Les exemples qui suivent illustrent le procédé de l'invention, dans le cas d'une mise en oeuvre à pression atmosphérique (exemples 1 à 14) et dans le cas d'une mise en oeuvre à pression réduite (exemples 15 à 18).

Comme ces exemples l'illustreront, le procédé de l'invention permet de synthétiser des molécules possèdant les formules suivantes :

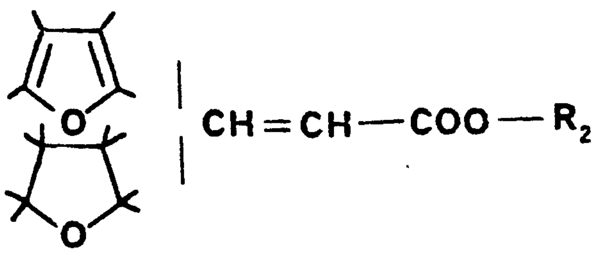

où $R_2$ est un radical ou un cycle hydrocarboné,

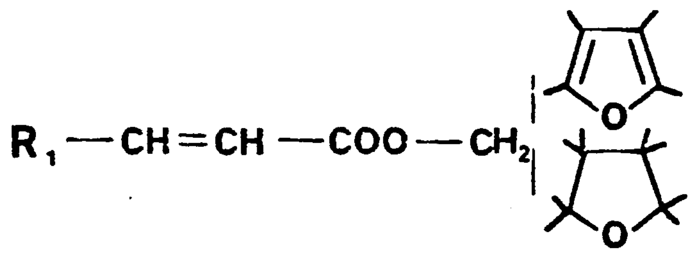

où $R_1$ est un hydrogène ou un radical ou un cycle hydrocarboné,

et 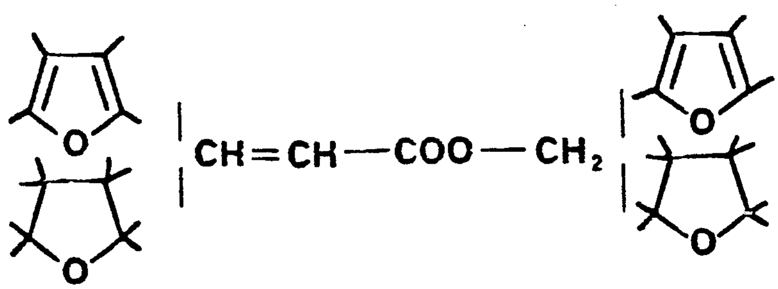

$$R_1-CH=CH-COO-R_2$$

dans laquelle $R_1$ est un hydrogène ou un radical ou cycle hydrocarboné, et $R_2$ un radical hydrocarboné à longue chaîne, à nombre de carbone ⩾ 8 ou un radical ou un cycle hydrocarboné comportant entre 1 et 5 fonctions alcool.

EXEMPLE 1 : Synthèse directe du furfurylidène-2

acétate de propyle à partir du furfural et de diethylphosphono-acétate d'éthyle en présence de carbonate de potassium dans le propanol.

On place dans un réacteur de 100 ml, 0,02 mole de furfural, 0,08 mole de carbonate de potassium, 0,025 mole de diethylphosphono-acétate d'éthyle dans 0,5 mole de propanol.

Le milieu réactionnel est agité pendant 3 heures à une température de 25° C.

A la fin de la réaction, la phase solide est séparée par simple filtration. La phase organique est réduite au rotavapor. Le brut réactionnel obtenu est purifié sur colonne de gel de silice "MERCK" avec un mélange éther/hexane comme éluant.

On obtient une molécule qui peut être désignée sous le nom chimique de "furfurylidène-2-acétate de propyle" dont la formule est :

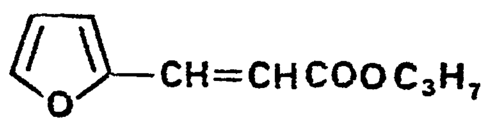

Ce corps est obtenu avec un rendement de 83 % et a été identifié par ses caractéristiques spectroscopiques en résonance magnétique du proton et du carbone 13, en infra-rouge et en ultra-violet. On a pu constater que, comme dans tous les exemples qui suivent, c'est l'ester éthylénique type E qui est obtenu préférentiellement (avec une proportion supérieure à 90 %).

<u>EXEMPLE 2</u> : Synthèse directe du furfurylidène-2 acétate de furfuryle à partir de furfural et de diethylphosphono-acétate d'éthyle en présence de carbonate de potassium dans l'alcool furfurylique.

On place dans un réacteur de 100 ml, 0,03 mole de furfural, 0,07 mole de carbonate de potassium, 0,035 mole de diethylphosphono-acétate d'éthyle dans 0,25 mole d'alcool furfurylique.

Le milieu réactionnel est agité pendant 4 heures à une température de 60° C.

A la fin de la réaction, la phase solide est séparée par simple filtration. La phase organique est réduite au rotavapor. Le brut réactionnel obtenu est purifié sur colonne de gel de silice "MERCK" avec un mélange éther/hexane comme

éluant.

On obtient une molécule qui peut être désignée sous le nom chimique de "furfurylidène-2 acétate de furfuryle" dont la formule est :

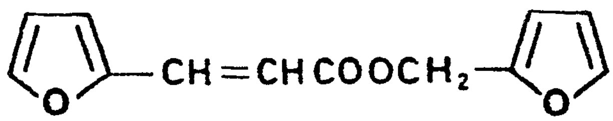

ce corps est obtenu pur avec un rendement de 81 % et a été identifié par ses caractéristiques spectroscopiques en résonance magnétique du proton et du carbone 13, en infra-rouge et en ultra-violet.

EXEMPLE 3 : Synthèse directe du furfurylidène-2 acétate de tétrahydrofurfuryle à partir de tétrahydrofurfural et de diméthylphosphono-acétate de méthyle en présence de carbonate ce cesium dans l'alcool tétrahydrofurfurylique.

On place dans un réacteur de 100 ml, 0,02 mole de tétrahydrofurfural, 0,06 mole de carbonate de cesium, 0,025 mole de diméthylphosphono-acétate de méthyle dans 0,25 mole d'alcool tétrahydrofurfurylique.

Le milieu réactionnel est agité pendant 5 heures à une température de 50° C.

A la fin de la réaction, la phase solide est séparée par simple filtration. La phase organique est réduite au rotavapor. Le brut réactionnel obtenu est purifié sur colonne de gel de silice "MERCK" avec un mélange éther/hexane comme éluant.

On obtient une molécule qui peut être désignée sous le nom chimique de "furfurylidène-2 acétate de tétrahydrofurfuryle" dont la formule est :

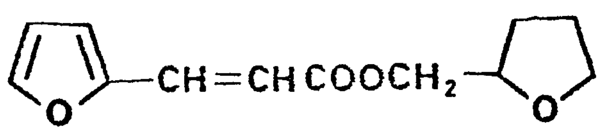

Ce corps est obtenu pur avec un rendement de 84 % et a été identifié par ses caractéristiques spectroscopiques en résonance magnétique du proton et du carbone 13, en infra-rouge et en ultra-violet.

EXEMPLE 4 : Synthèse directe du furfurylidène-2 acétate de furfuryle à partir de furfural et de diéthylphosphono-acétate d'éthyle en présence de carbonate de rubidium

dans l'alcool furfurylique.

On place dans un réacteur de 100 ml, 0,02 mole de furfural, 0,05 mole de carbonate de rubidium, 0,03 mole de diéthylphosphono-acétate d'éthyle dans 0,2 mole d'alcool furfurylique.

Le milieu réactionnel est agité pendant 3 heures à une température de 65° C.

A la fin de la réaction, la phase solide est séparée par simple filtration. La phase organique est réduite au rotavapor. Le brut réactionnel obtenu est purifié sur colonne de gel de silice "MERCK" avec un mélange éther/hexane comme éluant.

On obtient une molécule qui peut être désignée sous le nom chimique de "furfurylidène-2 acétate de furfuryle" dont la formule est déjà représentée dans l'exemple n° 2.

Ce corps est obtenu pur avec un rendement de 87 % et a été identifié par ses caractéristiques spectroscopiques en résonance magnétique du proton et du carbone 13, en infra-rouge et en ultra-violet.

EXEMPLE 5 : Synthèse directe du furfurylidène-2 acétate de butyle à partir de furfural et de diméthylphosphono-acétate de méthyle en présence de bicarbonate de potassium dans le butanol.

On place dans un réacteur de 100 ml, 0,3 mole de furfural, 0,08 mole de bicarbonate de potassium, 0,035 mole de diméthylphosphono-acétate de méthyle dans 0,4 mole de butanol.

Le milieu réactionnel est agité pendant 3 heures à une température de 70° C.

A la fin de la réaction, la phase solide est séparée par simple filtration. La phase organique est réduite au rotavapor. Le brut réactionnel obtenu est purifié sur colonne de gel de silice "MERCK" avec un mélange éther/hexane comme éluant.

On obtient une molécule qui peut être désignée sous le nom chimique de "furfurylidène-2 acétate de

butyle" dont la formule est :

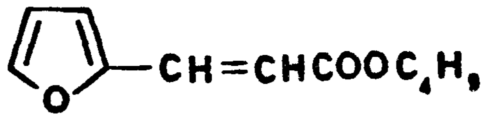

Ce corps est obtenu pur avec un rendement de 80 % et a été identifié par ses caractéristiques spectroscopiques en résonance magnétique du proton et du carbone 13, en infra-rouge et en ultra-violet.

EXEMPLE 6 : Synthèse directe de furfurylidène-2 acétate d'octyle à partir de furfural et de diéthylphosphono-acétate d'éthyle en présence de carbonate de potassium dans l'octanol-1.

On place dans un réacteur de 100 ml, 0,02 mole de furfural, 0,05 mole de carbonate de potassium, 0,025 mole de diéthylphosphono-acétate d'éthyle dans 0,25 mole d'octanol-1.

Le milieu réactionnel est agité pendant 5 heures à une température de 60° C.

A la fin de la réaction, la phase solide est séparée par simple filtration. La phase organique est réduite au rotavapor. Le brut réactionnel obtenu est purifié sur colonne de gel de silice MERCK avec un mélange éther/hexane comme éluant.

On obtient une molécule qui peut être désignée sous le nom chimique de "furfurylidène-2 acétate d'octyle" dont la formule est :

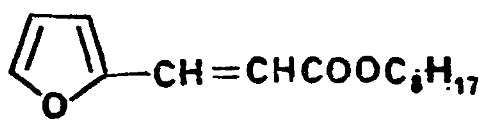

Ce corps est obtenu pur avec un rendement de 78 % et a été identifié par ses caractéristiques spectroscopiques en résonance magnétique du proton et du carbone 13, en infra-rouge et en ultra-violet.

EXEMPLE 7 : Synthèse directe du furfurylidène-2 acétate d'hexadecyle à partir de furfural et de diéthylphosphono-acétate d'éthyle en présence de carbonate de potassium dans l'hexadécanol-1.

On place dans un réacteur de 100 ml, 0,02 mole de furfural, 0,06 mole de carbonate de potassium, 0,025 mole de diéthylphosphono-acétate d'éthyle dans 0,25 mole d'hexadé-

canol-1.

Le milieu réactionnel est agité pendant 6 heures à une température de 70° C.

A la fin de la réaction, la phase solide est séparée par simple filtration. La phase organique est réduite au rotavapor. Le brut réactionnel obtenu est purifié sur colonne de gel de silice "MERCK" avec un mélange éther/hexane comme éluant.

On obtient une molécule qui peut être désignée sous le nom chimique de "furfurylidène-2 acétate d'hexadecyle" dont la formule est :

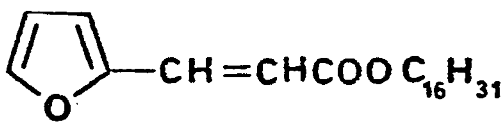

Ce corps est obtenu pur avec un rendement de 80 % et a été identifié par ses caractéristiques spectroscopiques en résonance magnétique du proton et du carbone 13, en infra-rouge et en ultra-violet.

<u>EXEMPLE 8</u> : Synthèse directe du nitro-5 furfurylidène-2 acétate de furfuryle à partir de nitro-5 furfural et de diéthylphosphono-acétate d'éthyle en présence de carbonate de potassium dans l'alcool furfurylique.

On place dans un réacteur de 100 ml, 0,03 mole de nitro-5 furfural, 0,04 mole de carbonate de potassium, 0,035 mole de diéthylphosphono-acétate d'éthyle dans 0,3 mole d'alcool furfurylique.

Le milieu réactionnel est agité pendant 2 heures à une température de 50° C.

A la fin de la réaction, la phase solide est séparée par simple filtration. La phase organique est réduite au rotavapor. Le brut réactionnel obtenu est purifié sur colonne de gel de silice "MERCK" avec un mélange éther/hexane comme éluant.

On obtient une molécule qui peut être désignée sous le nom chimique de "nitro-5 furfurylidène-2 acétate de furfuryle" dont la formule est :

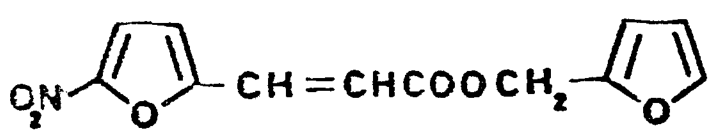

Ce corps est obtenu pur avec un rendement de 79 % et a été identifié par ses caractéristiques spectroscopiques en résonance magnétique du proton et du carbone 13, en infra-rouge et en ultra-violet.

<u>EXEMPLE 9</u> : Synthèse directe du cinnamate de butyle à partir de benzaldéhyde et de diéthylphosphono-acétate d'éthyle en présence de carbonate de potassium dans un mélange eau/butanol.

On place dans un réacteur de 100 ml, 0,02 mole de benzaldéhyde, 0,06 mole de carbonate de potassium, 0,03 mole de diéthylphosphono-acétate d'éthyle dans un mélange constitué par 0,1 mole d'eau et 0,3 mole de butanol.

Le milieu réactionnel est agité pendant 2 heures à une température de 60° C.

A la fin de la réaction, la phase solide est séparée par simple filtration. La phase organique est réduite au rotavapor. Le brut réactionnel obtenu est purifié sur colonne de gel de silice "MERCK" avec un mélange éther/hexane comme éluant.

On obtient une molécule qui peut être désignée sous le nom chimique de "cinnamate de butyle" dont la formule est :

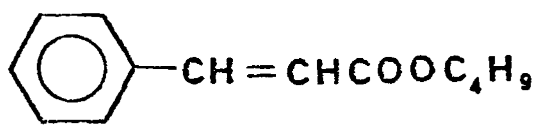

Ce corps est obtenu pur avec un rendement de 86 % et a été identifié par ses caractéristiques spectroscopiques en résonance magnétique du proton et du carbone 13, en infra-rouge et en ultra-violet.

<u>EXEMPLE 10</u> : Synthèse directe du cinnamate de furfuryle à partir de benzaldéhyde et de diéthylphosphono-acétate d'éthyle en présence de carbonate de potassium dans l'alcool furfurylique.

On place dans un réacteur de 100 ml, 0,03 mole de benzaldéhyde, 0,08 mole de carbonate de potassium, 0,04 mole de diéthylphosphono-acétate d'éthyle dans un mélange constitué par 0,1 mole de dioxanne et 0,2 mole d'alcool furfurylique.

Le milieu réactionnel est agité pendant 4 heures

à une température de 70° C.

A la fin de la réaction, la phase solide est séparée par simple filtration. La phase organique est réduite au rotavapor. Le brut réactionnel obtenu est purifié sur colonne de gel de silice "MERCK" avec un mélange éther/hexane comme éluant.

On obtient une nouvelle molécule qui peut être désignée sous le nom chimique de "cinnamate de furfuryle" dont la formule est :

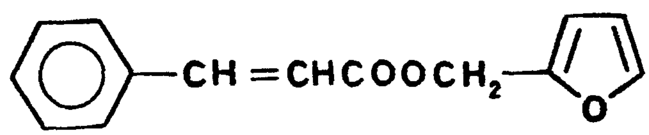

Ce corps est obtenu pur avec un rendement de 80 % et a été identifié par ses caractéristiques spectroscopiques en résonance magnétique du proton et du carbone 13, en infra-rouge et en ultra-violet.

<u>EXEMPLE 11</u> : Synthèse directe du p-hydroxycinnamate de butyle à partir de phydroxybenzaldéhyde et de diéthylphosphono-acétate d'éthyle en présence de carbonate de potassium dans le butanol.

On place dans un réacteur de 100 ml, 0,02 mole de p-hydroxybenzaldéhyde, 0,06 mole de carbonate de potassium, 0,025 mole de diéthylphosphono-acétate d'éthyle dans 0,3 mole de butanol.

Le milieu réactionnel est agité pendant 3 heures à une température de 50° C.

A la fin de la réaction, la phase solide est séparée par simple filtration. La phase organique est réduite au rotavapor. Le brut réactionnel obtenu est purifié sur colonne de gel de silice "MERCK" avec un mélange éther/hexane comme éluant.

On obtient une molécule qui peut être désignée sous le nom chimique de "p-hydroxycinnamate de butyle" dont la formule est :

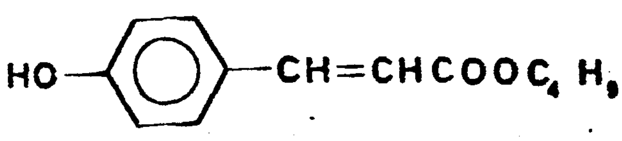

Ce corps est obtenu pur avec un rendement de 77 % et a été identifié par ses caractéristiques spectroscopiques en résonance magnétique du proton et du carbone 13, en infra-rouge et en ultra-violet.

EXEMPLE 12 : Synthèse directe du butanoate de furfuryle à partir de butanal et de diéthylphosphono-acétate d'éthyle en présence de carbonate de potassium dans l'alcool furfurylique.

On place dans un réacteur de 100 ml, 0,02 mole de butanal, 0,06 mole de carbonate de potassium, 0,025 mole de diéthylphosphono-acétate d'éthyle dans 0,25 mole d'alcool furfurylique.

Le milieu réactionnel est agité pendant 2 heures à une température de 40° C.

A la fin de la réaction, la phase solide est séparée par simple filtration. La phase organique est réduite au rotavapor. Le brut réactionnel obtenu est purifié sur colonne de gel de silice "MERCK" avec un mélange éther/hexane comme éluant.

On obtient une molécule qui peut être désignée sous le nom chimique de "butanoate de furfuryle" dont la formule est :

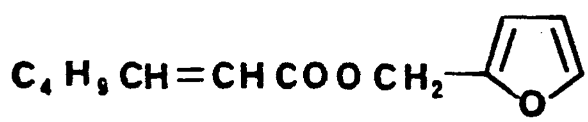

Ce corps est obtenu pur avec un rendement de 82 % et a été identifié par ses caractéristiques spectroscopiques en résonance magnétique du proton et du carbone 13, en infra-rouge et en ultra-violet.

EXEMPLE 13 : Synthèse directe de l'acrylate de furfuryle à partir de formaldéhyde aqueux à 35 % et de diéthylphosphono-acétate d'éthyle en présence de carbonate de potassium dans l'alcool furfurylique.

On place dans un réacteur de 100 ml, 0,02 mole de formaldéhyde en solution dans l'eau à 35 %, 0,06 mole de carbonate de potassium, 0,025 mole de diéthylphosphono-acétate d'éthyle dans 0,35 mole d'alcool furfurylique.

Le milieu réactionnel est agité pendant 3 heures à une température de 60° C.

A la fin de la réaction, la phase solide est séparée par simple filtration. La phase organique est réduite

au rotavapor. Le brut réactionnel obtenu est purifié sur colonne de gel de silice "MERCK" avec un mélange éther/hexane comme éluant.

On obtient une molécule qui peut être désignée sous le nom chimique de "acrylate de furfuryle" dont la formule est :

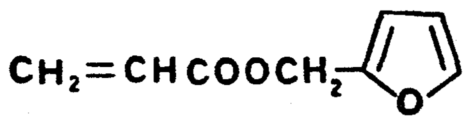

Ce corps est obtenu pur avec un rendement de 85 % et a été identifié par ses caractéristiques spectroscopiques en résonance magnétique du proton et du carbone 13, en infra-rouge et en ultra-violet.

<u>EXEMPLE 14</u> : Synthèse directe de l'acrylate de butyle à partir de paraformaldéhyde et de diéthylphosphono-acétate d'éthyle en présence de carbonate de potassium dans le butanol.

On place dans un réacteur de 100 ml, 0,03 mole de formaldéhyde, sous forme de paraformaldéhyde, 0,08 mole de carbonate de potassium, 0,035 mole de diéthylphosphono-acétate d'éthyle dans 0,3 mole de butanol.

Le milieu réactionnel est agité pendant 3 heures à une température de 60° C.

A la fin de la réaction, la phase solide est séparée par simple filtration. La phase organique est réduite au rotavapor. Le brut réactionnel obtenu est purifié sur colonne de gel de silice "MERCK" avec un mélange éther/hexane comme éluant.

On obtient une molécule qui peut être désignée sous le nom chimique de "acrylate de butyle" dont la formule est :

$$CH_2=CHCOOC_4H_9$$

Ce corps est obtenu pur avec un rendement de 80 % et a été identifié par ses caractéristiques spectroscopiques en résonance magnétique du proton et du carbone 13, en infra-rouge et en ultra-violet.

<u>EXEMPLE 15</u> : Synthèse directe du furfurylidène-

acétate de (méthyl-1, hydroxy-2) propyle à partir de furfural et de diméthylphosphono-acétate de méthyle en présence de carbonate de potassium dans le butanediol-2,3.

On place dans un réacteur de 250 ml, 0,5 mole de furfural, 1 mole de carbonate de potassium, 0,6 mole de diméthylphosphono-acétate de méthyle dans 3 moles de butanediol-2,3.

Le milieu réactionnel est agité sous un vide partiel de 200 mmHg (26 300 pascals) pendant 2 heures à une température de 60° C. L'alcool méthylique formé distille en continu pendant la réaction et est récupéré au moyen d'un appareil de "DEAN-STARK".

A la fin de la réaction, la phase solide est séparée par simple filtration. La phase organique est réduite au rotavapor. Le brut réactionnel obtenu est purifié sur colonne de gel de silice "MERCK" avec un mélange éther/hexane comme éluant.

On obtient une molécule qui peut être désignée sous le nom chimique de "furfurylidène-2 acétate de (méthyl-1, hydroxy-2) propyle" dont la formule est :

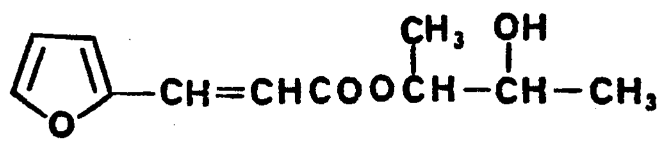

Ce corps est obtenu pur avec un rendement de 70 % et a été identifié par ses caractéristiques spectroscopiques en résonance magnétique du proton et du carbone 13, en infra-rouge et en ultra-violet.

<u>EXEMPLE 16</u> : Synthèse directe du furfurylidène-2 acétate de benzyle à partir de furfural et de diéthylphosphono-acétate d'éthyle en présence de carbonate de potassium dans l'alcool benzylique.

On place dans un réacteur de 250 ml, 0,5 mole de furfural, 0,7 mole de carbonate de potassium, 0,6 mole de diéthylphosphono-acétate d'éthyle dans 2 moles d'alcool benzylique.

Le milieu réactionnel est agité sous un vide partiel de 50 mmHg (6 575 pascals) pendant 3 heures à une température de 50° C. L'alcool éthylique formé distille en continu pendant la réaction et est récupéré au moyen d'un appareil

de "DEAN-STARK".

A la fin de la réaction, la phase solide est séparée par simple filtration. La phase organique est réduite au rotavapor. Le brut réactionnel obtenu est purifié sur colonne de gel de silice "MERCK" avec un mélange éther/hexane comme éluant.

On obtient une molécule qui peut être désignée sous le nom chimique de "furfurylidène-2 acétate de benzyle" dont la formule est :

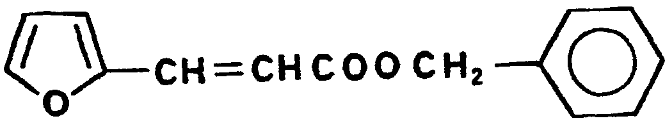

Ce corps est obtenu pur avec un rendement de 92 % et a été identifié par ses caractéristiques spectroscopiques en résonance magnétique du proton et du carbone 13, en infra-rouge et en ultra-violet.

<u>EXEMPLE 17</u> : Synthèse directe du tétrahydrofurfurylidène-2 acétate de furfuryle à partir de tétrahydrofurfural et de diéthylphosphono-acétate d'éthyle en présence de carbonate de potassium dans l'alcool furfurylique.

On place dans un réacteur de 250 ml, 0,5 mole de tétrahydrofurfural, 0,9 mole de carbonate de potassium, 0,6 mole de diéthylphosphono-acétate d'éthyle dans 2 moles d'alcool furfurylique.

Le milieu réactionnel est agité sous un vide partiel de 200 mmHg (26 300 pascals) pendant 3 heures à une température de 60° C. L'alcool éthylique formé distille en continu pendant la réaction et est récupéré au moyen d'un appareil de "DEAN-STARK".

A la fin de la réaction, la phase solide est séparée par simple filtration. La phase organique est réduite au rotavapor. Le brut réactionnel obtenu est purifié sur colonne de gel de silice "MERCK" avec un mélange éther/hexane comme éluant.

On obtient une molécule qui peut être désignée sous le nom chimique de "tétrahydrofurfurylidène-2 acétate de furfuryle" dont la formule est :

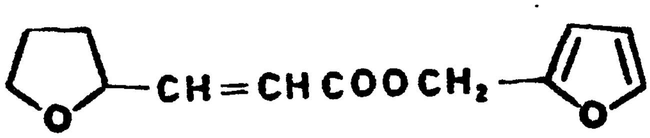

Ce corps est obtenu pur avec un rendement de 88 % et a été identifié par ses caractéristiques spectroscopiques en résonance magnétique du proton et du carbone 13, en infra-rouge et en ultra-violet.

<u>EXEMPLE 18</u> : Synthèse directe du furfurylidène-2 acétate de furfuryle à partir de furfural et de diéthylphosphono-acétate d'éthyle en présence de carbonate de potassium dans l'alcool furfurylique.

On place dans un réacteur de 250 ml, 0,5 mole de furfural, 0,9 mole de carbonate de potassium, 0,6 mole de diéthylphosphono-acétate d'éthyle dans 2 moles d'alcool furfurylique.

Le milieu réactionnel est agité sous un vide partiel de 200 mmHg (26 300 pascals) pendant 3 heures à une température de 60° C. L'alcool éthylique formé distille en continu pendant la réaction et est récupéré au moyen d'un appareil de "DEAN-STARK".

A la fin de la réaction, la phase solide est séparée par simple filtration. La phase organique est réduite au rotavapor. Le brut réactionnel obtenu est purifié sur colonne de gel de silice "MERCK" avec un mélange éther/hexane comme éluant.

On obtient une molécule qui peut être désignée sous le nom chimique de "furfurylidène-2 acétate de furfuryle" dont la formule est déjà représentée dans l'exemple n° 2.

Ce corps est obtenu pur avec un rendement de 92 % et a été identifié par ses caractéristiques spectroscopiques en résonance magnétique du proton et du carbone 13, en infra-rouge et en ultra-violet.

REVENDICATIONS

1/ - Procédé de transformation directe d'un aldéhyde 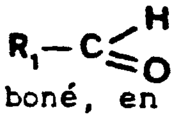 où $R_1$ est un hydrogène ou un radical ou cycle carboné, en un ester éthylénique

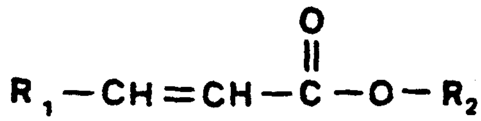

où $R_2$ est un radical ou un cycle hydrocarboné, ledit procédé étant du type dans lequel l'aldéhyde est mis en présence d'un phosphonate et d'un carbonate ou bicarbonate alcalin dans un solvant comportant une fonction alcool, et étant caractérisé en ce que, en vue d'obtenir un ester éthylénique dans lequel le radical $R_2$ est un radical hydrocarboné autre que méthyle ou éthyle, l'on utilise :

. d'une part, un phosphonate de méthyle ou d'éthyle

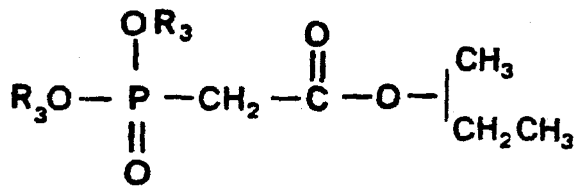

dans lequel $R_3$ est un radical ou un cycle hydrocarboné,

. d'autre part, un alcool ou un polyol $R_2OH$ comportant le radical hydrocarboné $R_2$ autre que méthyle ou éthyle, de l'ester éthylénique à obtenir.

2/ - Procédé de transformation directe selo la revendication 1, caractérisé en ce que le carbonate ou bicarbonate alcalin est mis en oeuvre en quantité telle que la proportion molaire de celui-ci par rapport à l'aldéhyde est supérieure à 1.

3/ - Procédé de transformation directe selc l'une des revendications 1 ou 2, caractérisé en ce que l'alco ou le polyol est mis en oeuvre en quantité telle que la propo tion molaire de celui-ci par rapport à l'aldéhyde est supérie re à 2.

4/ - Procédé de transformation directe sel

l'une des revendications 1, 2 ou 3, dans lequel on limite le taux d'hydratation du milieu réactionnel à une valeur comprise entre environ 0,1 et 5 moles d'eau par mole d'aldéhyde.

5/ - Procédé de transformation directe selon l'une des revendications 1, 2, 3 ou 4, dans lequel on utilise un phosphonate dans lequel le radical $R_3$ est un radical méthyle ou éthyle.

6/ - Procédé selon l'une des revendications 1, 2, 3, 4 ou 5, caractérisé en ce que la réaction de transformation directe est réalisée à une pression comprise entre 10 et 200 mm Hg (1 315 pascals et 26 300 pascals).

7/ - Procédé selon l'une des revendications 1, 2, 3, 4, 5 ou 6, en vue de la transformation directe d'aldéhyde furannique ou tétrahydrofurannique pour obtenir un ester éthylénique dont le radical $R_1$ possède un cycle furannique

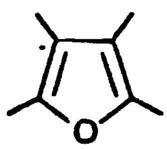

ou tétrahydrofurannique

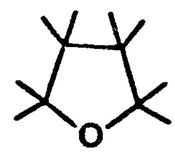

8/ - Procédé de transformation directe selon l'une des revendications 1, 2, 3, 4, 5 ou 6 ou 7, dans lequel on utilise l'alcool furfurylique ou tétrahydrofurfurylique pour obtenir un ester éthylénique dont le radical $R_2$ est un cycle furannique ou tétrahydrofurannique.

9/ - Procédé selon les revendications 7 et 8 prises ensemble en vue de la transformation directe d'aldéhyde furannique ou tétrahydrofurannique pour obtenir un ester éthylénique ayant deux cycles furanniques ou tétrahydrofuranniques, dans lequel on utilise l'alcool furfurylique ou tétrahydrofurfurylique.

10/ - Procédé selon l'une des revendications 1, 2, 3, 4, 5 ou 6, caractérisé en ce que l'on utilise un polyol dans lequel le radical $R_2$ contient entre 1 et 5 fonctions alcool.

11/ - Procédé selon l'une des revendications 1, 2, 3, 4, 5 ou 6, caractérisé en ce que l'on utilise un alcool gras possédant un radical $R_2$ à longue chaîne à nombre de carbones $\geqslant$ 8, en vue d'obtenir un ester éthylénique comportant une chaîne lipophile.

Office européen des brevets

**0140400**

Numéro de la demande

## RAPPORT DE RECHERCHE EUROPEENNE

EP 84 20 1167

### DOCUMENTS CONSIDERES COMME PERTINENTS

| Catégorie | Citation du document avec indication, en cas de besoin, des parties pertinentes | Revendication concernee | CLASSEMENT DE LA DEMANDE (Int. Cl.4) |
|---|---|---|---|
| D,A | EP-A-0 075 336 (AGRIFURANE)<br>* En entier *<br>----- | 1-11 | C 07 B 41/12<br>C 07 D 307/54<br>C 07 D 307/42<br>C 07 D 307/71<br>C 07 C 67/343 |
| | | | **DOMAINES TECHNIQUES RECHERCHES (Int. Cl.4)** |
| | | | C 07 B 41/00<br>C 07 C 67/00<br>C 07 D 307/00 |

Le présent rapport de recherche a été établi pour toutes les revendications

| Lieu de la recherche | Date d'achèvement de la recherche | Examinateur |
|---|---|---|
| LA HAYE | 19-12-1984 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82